(19) **Europäisches Patentamt / European Patent Office / Office européen des brevets**

(11) **EP 4 324 849 A1**

(12) ## EUROPEAN PATENT APPLICATION

(43) Date of publication:
**21.02.2024 Bulletin 2024/08**

(21) Application number: **22191026.8**

(22) Date of filing: **18.08.2022**

(51) International Patent Classification (IPC):
**C07K 16/28** $^{(2006.01)}$

(52) Cooperative Patent Classification (CPC):
**C07K 16/2803; A61K 47/6831; A61K 47/6849;**
A61K 2039/505; A61P 35/00; C07K 2317/24;
C07K 2317/34; C07K 2317/92

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Emergence Therapeutics AG**
  **47059 Duisburg (DE)**
- **Université d'Aix Marseille**
  **13007 Marseille (FR)**
- **INSTITUT NATIONAL DE LA SANTE ET DE LA RECHERCHE MEDICALE - INSERM**
  **75013 Paris (FR)**
- **Centre National de la Recherche Scientifique (CNRS)**
  **75016 Paris (FR)**
- **Institut Jean Paoli & Irène Calmettes**
  **13009 Marseille (FR)**

(72) Inventors:
- **ELANDS, Jack**
  **1190 Forest (BE)**
- **LHOSPICE, Florence**
  **29770 Primelin (FR)**
- **PRÉVILLE, Xavier**
  **06330 Roquefort les Pins (FR)**
- **OLIVE, Daniel**
  **13009 Marseille (FR)**
- **LOPEZ, Marc**
  **13009 Marseille (FR)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

(54) **HUMANIZED ANTI-NECTIN-4 ANTIBODIES**

(57) The present invention relates to humanized antibodies having specificity to Nectin-4 and uses thereof.

**EP 4 324 849 A1**

**Description**

The present invention relates to humanized antibodies having specificity to Nectin-4 and uses thereof.

**Background information**

Nectins are adhesion molecules that help organize epithelial and endothelial junctions and serve as receptors for the entry of the herpes simplex virus, measle virus and poliovirus.

Nectins belong to the immunoglobulin superfamily and are homologs of the poliovirus receptor (PVR / CD155), and for this, have also been called poliovirus receptor bound proteins (PRR). To date, 5 members have been described PVR / CD155, Nectin-1 / PRR1 / CD111, Nectin-2 / PRR2 / CD112, Nectin-3 / PRR3 and Nectin-4 / PRR4. Their ectodomain is made up of three immunoglobulin-like (Ig) -type V, C, C domains that share between 30 and 55% identity in their amino acid sequences.

Expression of Nectin / PRR molecules is generally extensive in tissues, including hematopoietic, neuronal, endothelial and epithelial cells, with the exception of Nectin-3 and -4, which exhibit more restricted expression profiles.

Nectin-4 is a particularly interesting target. It is expressed during fetal development, but its expression decreases and is very restricted in adult tissues in comparison to that of other members of the Nectin family. Nectin-4 is a tumor associated antigen in 83 % of bladder cancers, 78 % of breast cancers (mainly triple negative and ERBB2+), 71 % Pancreatic cancers, 55 % of lung cancers, 57% of ovarian cancers, 59% of head and neck cancers, and 55% of esophageal cancers.

Expression of Nectin-4 in these pathologies is associated with poor prognosis, probably because of the ability of Nectin-4 to confer to tumor cells *in vitro,* higher capacities of migration, proliferation and formation of metastases.

In normal tissues, Nectin-4 is only detected in skin, salivary glands, urinary bladder and esophagus. The recent approval by heath authorities of Enfortumab vedotin for the 2nd line treatment of advanced urothelial cancer has completed the validation of Nectin-4 as a target for the treatment of cancer.

**Detailed Description of the Invention**

The present invention relates to humanized antibodies having specificity to Nectin-4, antigen-binding fragments thereof as well as uses thereof.

In particular, the present invention provides humanized antibodies that derive from the monoclonal anti-Nectin-4 antibody 5A12.2 mAb.

The humanized variants derived from 5A12.2 which can be provided with high yields represent a new way to improve therapeutic index of Nectin-4 positive cancer treatment combined with reduced skin toxicity and high anti-tumor selectivity and efficacy.

Surprisingly, the antibodies of the invention can be provided with significantly higher yield compared to non-humanized antibodies. This could be shown for small as well as larger production approaches. Such reproducible production in high yields greatly facilitates pharmaceutical use.

A first aspect of the invention relates to a monoclonal antibody or an antigen-binding fragment thereof which binds to a discontinuous epitope on Nectin-4 consisting of amino acids L81, H83, Y86, G87, H89, S91, P92 and E95 of SEQ ID NO: 1 as determined using Deep Mutational Scanning (DMS). DMS is known to the person skilled in the art. Basically, every possible amino acid change in a given protein is first synthesized. The activity of each of these protein variants is assayed in parallel using barcodes for each variant. By comparing the activity to the wild-type protein, the effect of each mutation is identified. One detailed example of DMS is given herein below.

Preferably, the inventive antibodies has a better avidity for their dimeric epitope on Nectin-4 than the prior art antibody HA22. HA22 is a human monoclonal anti-Nectin-4 antibody described in WO2012/047724, the content of which is herein incorporated by reference. "Avidity" as herein described describes the measure of overall or accumulated strength of a protein-protein complex, i.e. the total strength of all the noncovalent interactions between an antibody binding to its ligand/s. It is determined by three parameters: the binding affinity of the complex, the valency of the proteins and the structural arrangement of the proteins in the complex.

The antibodies of the invention are monoclonal antibodies (mAb) or monoclonal antibody fragments characterized by a specific amino acid sequence. If not indicated differently, the term "monoclonal" refers to a single species, i.e. single amino acid composition of antibodies or antibody fragments.

The antigen-binding site of an inventive antibody comprises heavy chain variable domains/regions (VH) and/or antibody light chain variable domains/regions (VL), or pairs of VH/VL. The variable domains/region denote each of the pair of light and heavy chains, which is involved directly in binding the antibody to the antigen. The variable domain of a heavy chain is abbreviated as "VH" and the variable domain of a light chain is abbreviated as "VL".

The term "antigen-binding site" denotes the region(s) of an antibody molecule to which a ligand (e.g. the antigen, i.e. Nectin-4, or antigen fragment of it) actually binds and which is derived from an antibody.

An antigen-binding site of an antibody according to the invention can contain six complementarity determining regions (CDRs) which contribute in varying degrees to the affinity of the binding site for the antigen. There are three heavy chain variable domain CDRs (CDR-H1, CDR-H2 and CDR-H3) and three light chain variable domain CDRs (CDR-L1, CDR-L2 and CDR-L3). Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

According to the present invention, a VH region or the CDRs thereof alone may constitute a complete antigen-binding site. In certain embodiments, the antibody comprises a VH region or the CDRs thereof as defined herein alone. In other embodiments, the antibody comprises a VH region or the CDRs thereof as defined herein together with a VL region or the CDRs thereof, particularly with a VL region or the CDRs thereof as defined herein.

The position of CDRs within a VH or VL region may be defined according to Kabat, et al., Sequences of Proteins of Immunological Interest, 5th Ed. Public Health Service, National Institutes of Health, Bethesda, MD (1991) or the IMGT numbering system, both of which are known to the person skilled in the art. The IMGT numbering has been defined to compare the variable domains whatever the antigen receptor, the chain type, or the species (Lefranc M.-P., "Unique database numbering system for immunogenetic analysis" Immunology Today, 18, 509 (1997); Lefranc M.-P., "The IMGT unique numbering for Immunoglobulins, T cell receptors and Ig-like domains" The Immunologist, 7, 132-136 (1999)).

A further aspect of the present invention relates to a humanized monoclonal antibody or antigen-binding fragment thereof which binds to Nectin-4 comprising

(a) a variable heavy chain (VH) region comprising an amino acid sequence according to any one of SEQ ID NOs: 2-5, or an amino acid sequence having an identity thereto of at least 85%, at least 90%, at least 95% or at least 99%, and including complementarity-determining regions (CDRs) CDR-H1 according to SEQ ID NO: 10 or 11, CDR-H2 according to SEQ ID NO: 12 or 13 and CDR-H3 according to SEQ ID NO: 14, and

(b) a variable light chain (VL) region comprising an amino acid sequence according to any one of SEQ ID NOs: 6-9, or an amino acid sequence having an identity thereto of at least 85%, at least 90%, at least 95% or at least 99%,

and including CDR-L1 according to SEQ ID NO: 15 or 16, CDR-L2 according to SEQ ID NO: 17 or 18 and CDR-L3 according to SEQ ID NO: 19.

Specific amino acid sequences represented by SEQ ID NO:s used herein are provided in the attached sequence listing. SEQ ID NO: 10-19 define the six CDR sequences of the inventive humanized antibody according to the Kabat numbering system. If not stated otherwise, the Kabat system is used herein.

According to the present invention, a substitution of 1 or 2 amino acids in these CDR sequences is possible. In particular embodiments, a conservative amino acid substitution is preferable, i.e. a substitution of an amino acid by another amino acid with similar biochemical properties, for example a substitution of an aliphatic amino acid, e.g. Gly, Ala, Val, Leu or Ile, for another aliphatic amino acid, a basic amino acid, e.g. His, Lys or Arg, against another basic amino acid, an acidic amino acid or an amide thereof, e.g. Asp, Glu, Asn or Gln, against another acidic amino acid or an amide thereof, an aromatic amino acid, e.g. Phe, Tyr or Trp, against another aromatic amino acid, or a hydroxy or sulfur containing amino acid, e.g. Ser, Thr, Met or Cys, against another hydroxy or sulfur containing amino acid.

An "antigen-binding fragment" of an antibody refers to a molecule comprising a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments. The term also encompasses a fusion protein, e.g. a fusion protein with a non-immunoglobulin peptide or polypeptide, and a conjugate with a non-proteinaceous structure, e.g. a label or a toxin. The terms "antigen-binding fragment of an antibody", "antigen-binding fragment thereof", "fragment of an antibody" or "fragment thereof" may be used interchangeably herein.

The inventive antibody or the antigen-binding fragment thereof may be mono- or multivalent, i.e. it may comprise a single antigen-binding site or multiple antigen-binding sites. For example, Fab fragments have single antigen-binding site, antibodies of the IgG class or Fv or scFv fragments have two antigen-binding sites and antibodies of the IgM class have 5 antigen-binding sites. The term "antibody" also encompasses hetero-specific antibodies, e.g. hetero-bispecific antibodies, which have different antigen-binding sites, particularly antibodies, which are directed to two different epitopes on the antigen. As used herein, "epitope" is a region of an antigen that is bound by an antibody. The term "epitope" includes any polypeptide determinant capable of specific binding to an antibody.

According to another preferred embodiment, the humanized antibody or antigen-binding fragment thereof comprises

(a) a VH region comprising an amino acid sequence according to SEQ ID NO:2-5, and

(b) a VL region comprising an amino acid sequence according to SEQ ID NO: 6-9.

More particularly, the antibody or antigen-binding fragment thereof may comprise

(i) a VH region comprising an amino acid sequence according to SEQ ID NO: 2 or an amino sequence having an identity thereof of at least 85%, at least 90%, at least 95% or at least 99%, and
a VL region comprising an amino acid sequence according to SEQ ID NO: 6 or an amino sequence having an identity thereof of at least 85%, at least 90%, at least 95% or at least 99%,
(ii) a VH region comprising an amino acid sequence according to SEQ ID NO: 3, or an amino sequence having an identity thereof of at least 85%, at least 90%, at least 95% or at least 99%, and
a VL region comprising an amino acid sequence according to SEQ ID NO: 7 or an amino sequence having an identity thereof of at least 85%, at least 90%, at least 95% or at least 99%,
(iii) a VH region comprising an amino acid sequence according to SEQ ID NO:4 or an amino sequence having an identity thereof of at least 85%, at least 90%, at least 95% or at least 99%, and
a VL region comprising an amino acid sequence according to SEQ ID NO: 8, or an amino sequence having an identity thereof of at least 85%, at least 90%, at least 95% or at least 99%,
(iv) a VH region comprising an amino acid sequence according to SEQ ID NO: 5, or an amino sequence having an identity thereof of at least 85%, at least 90%, at least 95% or at least 99%, and

a VL region comprising an amino acid sequence according to SEQ ID NO: 9, or an amino sequence having an identity thereof of at least 85%, at least 90%, at least 95% or at least 99%.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, Alignment for purposes of determining percent amino acid sequence identity can be achieved in various ways that are within the skill in the art, for instance, using publicly available computer software such as BLAST.

The antibody of the invention may be a chimeric antibody, a multispecific antibody, in particular a bispecific antibody, a human antibody, a humanized antibody or an antigen-binding fragment thereof.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" which binds two different epitopes

The term "humanized antibody" or "humanized version of an antibody" refers to antibodies for which both heavy and light chains are humanized as a result of antibody engineering. A humanized chain is typically a chain in which the V-region amino acid sequence has been changed so that, analyzed as a whole, is closer in homology to a human germline sequence than to the germline sequence of the species of origin. For example, a murine CDR may be grafted into the framework region of a human antibody to prepare the "humanized antibody." See, e.g., Riechmann, L., et al., Nature 332 (1988) 323-327; and Neuberger, M. S., et al., Nature 314 (1985) 268-270. Other forms of humanized antibodies encompassed by the present invention are those in which the constant region has been additionally modified or changed from that of the original antibody to generate the properties according to the invention. Humanization assessment is based on the resulting amino acid sequence and not on the methodology per se.

The antibody of the present invention may be of any suitable class. The term "class" refers to the type of constant domain or constant region possessed by its heavy chain. As used herein, "constant domain" or "constant region" denotes the sum of the domains of an antibody other than the variable region. The constant region is not directly involved in binding of an antigen, but exhibits various effector functions. The antibody may be of any of the five major classes of antibodies: IgA, IgD, IgE, IgG, and IgM, or any subclass thereof (isotype), e.g., IgG1, IgG2, IgG3, IgG4, IgA1, and IgA2. The heavy chain constant domains that correspond to the different classes of immunoglobulins are called a, $\delta$, $\epsilon$, $\gamma$ and $\mu$, respectively. According to the present invention, an antibody of the class IgG, IgA or IgM or a fragment thereof is particularly suitable.

According to a preferred embodiment, an antibody of the invention is selected from class IgG, e.g. of subclass IgG1, IgG2, IgG3 of IgG4, of class IgM, of class IgA or an antigen-binding fragment thereof.

As used herein, the terms "binding" and "specific binding" refer to the binding of the inventive antibody or fragment thereof to an epitope of the Nectin-4 antigen. The measure of the binding strength of an antibody is referred to as affinity. Methods for determining such a binding and/or affinity using in vitro assays are known to the person skilled in the art. According to the present invention, detection with flow cytometry, immuno-histochemistry and/or fluorescence are described and in particular preferred herein.

The affinity of the binding of an antibody to an antigen is defined by the terms Ka (rate constant for the association of the antibody from the antibody/antigen complex), KD (dissociation constant), and $K_{dis}$ (kD/ka), i.e. the term "Ka", as used herein, refers to the association rate of a particular antibody-antigen interaction, whereas the term "Kd" refers to the dissociation rate of a particular antibody-antigen interaction. Such values for an antibody can also be determined using methods well established in the art and also described herein.

Antibodies according to the invention and antigen-binding fragments thereof preferably show a dissociation constant KD of at least 3.5, preferably at least 4.0, more preferably at least 4.2 and most preferably at least 4.5 (nM).

Of course, a specific binding to human Nectin-4 expressed by tumors is preferred. Thus, the antibodies provided herein show preferably specific binding to Nectin-4 and no essential or no cross reactivity to other proteins, in particular to proteins of the human Nectin-family such as Nectin-1.

The antibody or the antigen-binding fragment thereof may be produced in a suitable host cell comprising a nucleic acid molecule, e.g. a DNA molecule, encoding an antibody VH region, or an antibody VL region, or encoding a complete antibody or an antibody fragment, or a vector or vector system, i.e. a plurality of vectors, comprising said nucleic acid molecule(s), preferably in operative linkage with an expression control sequence, particularly with a heterologous expression control sequence. A method for providing the inventive antibodies is described herein. The host cell may be any known host cell for producing antibodies or antibody fragments, e.g. a prokaryotic cell such as an E. coli cell, a yeast cell, an insect cell or a mammalian cell, e.g. a CHO cell or a hybridoma cell.

According to a further embodiment, inventive antibodies or antigen-binding fragments thereof comprise a labeling group and/or an effector group coupled to the antibody or antigen-binding fragment. The labeling group may be, for example, a dye, a paramagnetic, radioactive or fluorogenic group that is detectable upon imaging. A preferred effector group is a therapeutic group, in particular a cytotoxic agent, such as chemotherapeutically active agents, drugs, anti-inflammatory agents, radioactive isotopes, toxins such as topoisomerase poisons, enzymes and fragments thereof such as nucleolytic enzymes, growth inhibitory agents, antibiotics as wells as all suitable anticancer and antitumor agents known to the person skilled in the art. Especially preferred is the topoisomerase poison Camptothecin and derivatives and/or structural analogues thereof like Exatecan as well as derivatives thereof like Deruxtecan.

A further aspect of the invention relates to an antibody-drug conjugate (ADC), comprising a humanized antibody or antigen-binding fragment thereof as herein described and a drug conjugated to a reactive amino acid residue on the antibody or antigen-binding fragment, e.g., an amino acid residue having a side chain comprising an amino, hydroxy or thiol group, or a reactive group in the antibody glycan structure. Preferably, the drug is conjugated to a reactive thiol group in the side chain of a cysteine residue on the antibody or antigen-binding fragment thereof.

The drug of the antibody conjugate of the invention is preferably a topoisomerase I inhibitor, auristatin or maytansinoid.

A topoisomerase I inhibitor is a compound, which is capable of forming a ternary complex with topoisomerase I and DNA, thereby preventing DNA re-ligation and introducing DNA strand breaks in the cellular genome. The topoisomerase I inhibitor may be e.g., selected from camptothecin or analogs thereof, indenoisoquinolines and indolocarbazoles. In a particular embodiment, the topoisomerase-I-inhibitor is camptothecin or an analog thereof, i.e. a compound comprising the pentacyclic basic structure of camptothecin and modified substituents optionally resulting in the presence of a further ring. Specific examples are camptothecin, topotecan, irinotecan, SN-38, belotecan, exatecan including derivatives thereof such as deruxtecan, lurtotecan or atiratecan. In a particular preferred embodiment, the topoisomerase I inhibitor is exatecan. Exatecan is typically covalently conjugated to an antibody via its $NH_2$ group.

A particular preferred auristatin is monomethyl auristatin E (MMAE) which is a synthetic antineoplastic agent. MMAE inhibits cell division by blocking the polymerisation of tubulin.

Derivatives of maytansine are known as maytansinoids. Maytansinoids inhibit the assembly of microtubules by binding to tubulin at the rhizoxin binding site. Examples of maytansinoids are ansamitocin, Mertansine/emtansine (DM1) and Ravtansine/soravtansine (DM4).

In principle, the drug, e.g. exatecan, may be conjugated to any suitable position of the monoclonal antibody or antigen-binding fragment thereof, particularly to any position, which does not abolish the binding of the antibody to Nectin-4. For example, the topoisomerase-I-inhibitor, e.g. exatecan, may be conjugated to a reactive amino acid residue on the antibody, e.g. an amino acid residue having a side chain comprising an amino, hydroxy or thiol group, or a reactive group in the antibody glycan structure. In particular embodiments, drug, e.g. exatecan, is conjugated to a reactive thiol group in the side chain of an accessible cysteine residue on the antibody.

In certain embodiments, the antibody drug conjugate comprises has a molar drug-antibody/antibody fragment ratio (DAR) of greater than 1, i.e. more than one drug molecule is attached to an antibody/antibody fragment. Typically, the conjugate has a DAR of about 2:1 to about 16:1, particularly of about 4:1 to about 10:1 and more particularly of about 6:1 to about 8:1. The DAR may be calculated from a statistical distribution according to known methods.

In certain embodiments, the drug is conjugated to the antibody or antigen-binding fragment via a linker. In certain embodiments, the linker is a cleavable linker, i.e. a linker cleavable under physiological conditions, e.g. by physiological enzymes. Specific examples of cleavable linkers are peptide-based linkers, which may be subject to cleavage by a protease, or glycoside-based linkers, which may be subject to cleavage by a glycosidase.

In certain embodiments, the linker is a hydrophilic polysarcosine linker e.g. as described by Conilh et at. "Exatecan antibody drug conjugates based on a hydrophilic polysarcosine drug-linker platform". (Pharmaceuticals 14 (2021), 247). Further preferred linkers include linkers comprising at least one ethylene glycol unit, e.g. 1, 2, 3, 4, 5, 6, 7, 8, 9, or 10 or more ethylene glycol units, e.g. the linker of the antibody-drug conjugate MEDI7247, an mcc-triazole spacer-PEG7-x-Lys-PABC glycol linker from Trodelvy®, Further preferred linkers include oligopeptide, particularly di- to decapeptide, e.g. tetrapeptide sequences such as glycine-glycine-phenylalanine-glycine from Enhertu®. Further preferred linkers include highly polar spacers such as an acyl group, carbamoyl group and/or sulfamide group added to at least at least one ethylene glycol unit, e.g. 1, 2, 3, 4, 5, 6, 7, 8, 9, or 10 or more ethylene glycol units, e.g. the linker technology called HydraspaceTM.

The antibody drug conjugate of the present invention may be prepared by known methods.

The antibody or antigen-binding fragment thereof may be reacted with a linker-drug conjugate to obtain the antibody-drug conjugate. The linker-drug conjugate comprises a drug molecule, e.g. having attached thereto a suitable linker, wherein the linker comprises a reactive group capable of reacting with desired attachment positions on the antibody or antigen-binding fragment thereof. For attachment to cysteine residues, the linker-drug conjugate comprises a thiol-reactive group, e.g. a maleimide group.

A preferred embodiment of an antitumor agent relates to antitumor immune stimulating agents including but not restricted to toll-like receptor (TLR) agonists or stimulators of interferon genes (STING) pathways.

According to another preferred embodiment, an anti-inflammatory agent may be selected from group comprising steroids and corticosteroids such as glucocorticoids, e.g. cortisol and derivatives thereof, or mineralocorticoids such as aldosterone and derivatives thereof.

According to a further aspect of the invention, the antibody or antigen-binding fragment thereof is for use in medicine, particularly for therapeutic or diagnostic applications including in vitro and in vivo diagnostic applications.

The antibody or antigen-binding fragment thereof may be of use in a method of preventing and/or treating cancer and/or inflammatory disorders, wherein the cancer and/or inflammatory disorder is preferably associated with Nectin-4 overexpression.

The cancer to be prevented and/or treated may be any kind of cancer, wherein the term "cancer" is used herein to refer to proliferative diseases. The cancer to be prevented and/or treated according to the present invention is preferably selected from the group consisting of urothelial, endometrial, cervical, colorectal, liver, bladder, thyroid, breast, pancreatic, lung, ovarian head and neck and/or esophagus cancer.

Being specific for Nectin-4 expressed on tumors, the inventive antibodies and fragments thereof may be also used in diagnostics, for example being coupled to a labeling group as described above.

According to a further aspect, the antibody or antigen-binding fragment thereof as described herein may be used in a method of preventing and/or treating an inflammatory disorder, wherein the inflammatory disorder is preferably associated with Nectin-4 expression. Such treatment may be preferably combined with an anti-inflammatory agent known to the person skilled in the art. Preferred anti-inflammatory agents are described above.

Another aspect of the present invention is a combination of at least 2 different monoclonal antibodies or fragments as described herein.

Further, the present invention relates to a nucleic acid molecule, e.g. a DNA molecule, encoding an antibody VH region, or an antibody VL region, or encoding a complete antibody or an antibody fragment as indicated above, a vector or vector system, i.e. a plurality of vectors, comprising said nucleic acid molecule(s) as indicated above, preferably in operative linkage with an expression control sequence, particularly with a heterologous expression control sequence.

Furthermore, the invention relates to a cell comprising a nucleic acid molecule or a vector or vector system as described above. The term "vector," as used herein, refers to a nucleic acid molecule capable of propagating another nucleic acid to which it is linked. According to a preferred embodiment, the vector is an expression vector. An "expression vector" is a vector are capable of directing the expression of nucleic acids to which they are operatively linked. Vectors, in particular expression vectors, for the recombinant production of antibodies are well known in the art.

The cell may be a known host cell for producing antibodies or antibody fragments, e.g. a prokaryotic cell such as an E. coli cell, a yeast cell, an insect cell or a mammalian cell, e.g. a CHO cell or a hybridoma cell.

Still a further aspect of the present invention relates to a pharmaceutical composition comprising an antibody or antigen-binding fragment thereof as described herein. Typically, the antibody or antibody-fragment is administered as a pharmaceutical composition comprising the active agent and a pharmaceutically acceptable carrier or excipient. Examples of suitable carriers and excipients for formulating antibodies or antibody fragments include saline and aqueous buffer solutions and are well known in the art.

Depending on the stage and the severity of the disorder to be treated, the pharmaceutical composition may be administered once or several times during the course of the disorder. For example, it may be administered daily, each second day, two times weekly or weekly for a suitable period of time.

In certain embodiments, the pharmaceutical composition is administered parenterally, e.g. by subcutaneous, intramuscular or intravenous injection or by infusion. In further embodiments, the pharmaceutical composition may be administered locally, e.g. orally, nasally or pulmonally, for example as an aerosol.

The invention is further illustrated by the following figures and examples, which are not intended to limit the scope of the invention.

**Figures**

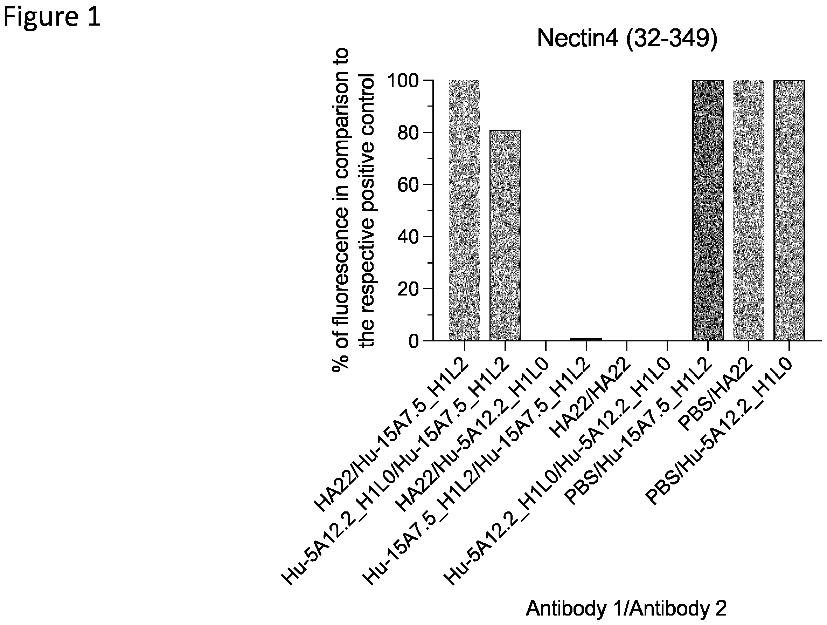

**Fig. 1: Evaluation of the compatibility of antibodies 5A12.2_H1L0, HA22 (Enfortumab) and 15A7.5_H1L2**
Yeasts expressing the Nectin-4 fragment 32-349 were incubated with 100 nM of unlabeled antibody 1 or PBS (as positive control). After 30 minutes incubation, 500 pM of antibody 2 was added for 30 minutes. Yeasts were then incubated with fluorescent reporters to detect by FACS Nectin-4 expression and antibody 2. Results are expressed as percentage of fluorescence to the respective positive control

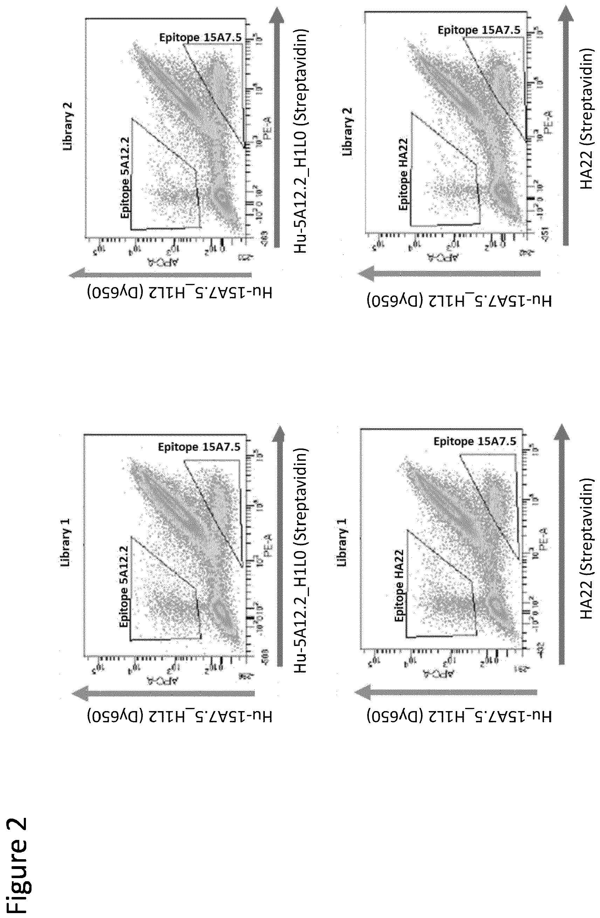

**Fig. 2: Libraries sorting by flow cytometry**
Two libraries covering the Nectin-4 sequence of interest were generated (left and right panels). Yeasts expressing Nectin-4 mono-mutants were incubated either with 100 pM of biotinylated 5A12.2_H1L0 and 100 pM of 15A7.5_H1L2 labelled with Dy650 (upper panels), or 100 pM of biotinylated HA22 and 100 pM of 15A7.5_H1L2 labelled with Dy650 (lower panels). Streptavidin PE was used to reveal biotinylated antibodies. After PBS washing, cells were analyzed and sorted on BD FACSAria III as indicated on the graphs. Single-stained yeasts by an antibody express Nectin-4 mono mutants that disrupt the epitope for the binding of the other antibody.

**Fig. 3: Positions showing a significant impact on the 5A12.2_H1L0 antibody when mutated.**
For any amino acid of the domain of interest, the higher the number of forbidden mutations, the more important the residue for the binding of 5A12.2_H1L0 antibody.

**Fig. 4: Positions showing a significant impact on the HA22 (Enfortumab) antibody when mutated.**
For any amino acid of the domain of interest, the higher the number of forbidden mutations, the more important the residue for the binding of HA22 antibody.

**Fig. 5: Structure reference of Nectin-4 (4FRW, pdb)**
Shown are pymol files. For each antibody, important amino acids of the epitope are indicated. Most important residues are underlined.

**Fig. 6: in vitro cytotoxic activity to SUM190PT and T47D tumor cells**
HA22 (Enfortumab, circles), Ch-5A12.2 (triangles) and Isotypic control (open squares) mAbs were coupled to $\alpha$-amanitin to generate antibody drug conjugates which cytotoxic activity of a dose range (left: 1 pg/mL - 15 $\mu$g/mL, right: 192 pg/mL - 15 $\mu$g/mL) was evaluated on SUM190PT (left) and T47D (right) Nectin-4-expressing cell lines. Viability (AlamarBlue assay) after a 5-days incubation period is reported. EC50 values were determined by GraphPad Prism 9 software using non-linear curve fitting (4 parameters).

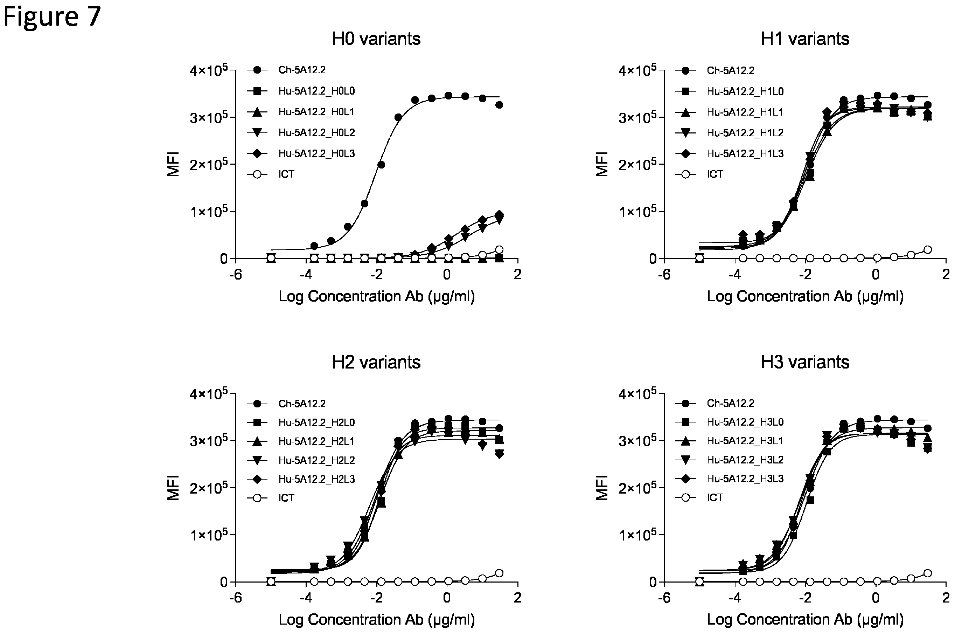

**Fig. 7: Apparent affinity of humanized variants to tumor cells. Detection by flow cytometry.**
T47D human tumor cells expressing Nectin-4 were numbered and incubated with a dose range (169 pg/mL - 30 $\mu$g/mL) of Ch-5A12.2 or indicated humanized variants. Numbers associated with H and L refer to the number of back mutations introduced. Cells were then stained with phycoerythrin conjugate goat anti human Fc antibody and analyzed by flow cytometry. Mean fluorescence intensities are reported. GraphPad Prism 9 software was used with non-linear curve fitting (4 parameters).

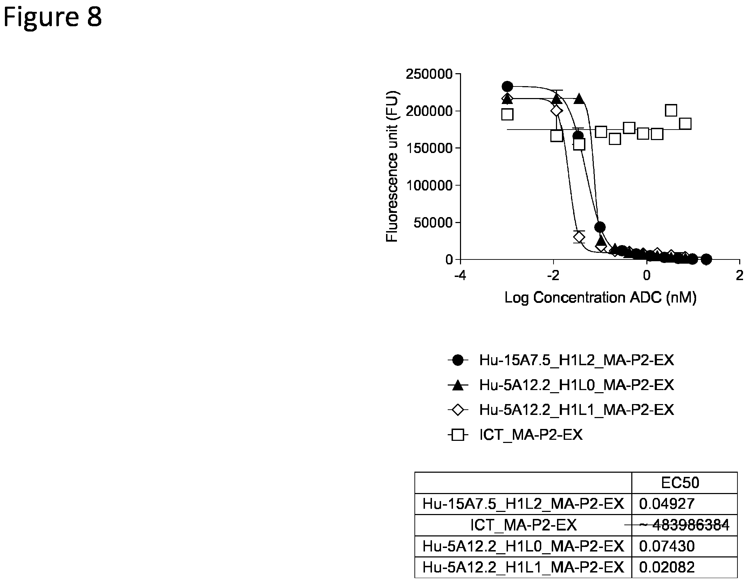

**Fig. 8: in vitro cytotoxic activity to HCT-116 transfectant expressing Nectin-4**
Hu-5A12.2_H1L0 (triangles), Hu-5A12.2_H1L1 (open diamonds), Hu-15A7.5_H1L2 (circles) and isotypic control (ICT, open squares) mAbs were coupled to exatecan to generate antibody drug conjugates which cytotoxic activity of a dose range (left: 1 pM - 18.94 nM) was evaluated on HCT-116 Nectin-4-expressing cell lines. Viability (MTT assay) after a 7-days incubation period is reported. EC50 values were determined by GraphPad Prism 9 software using non-linear curve fitting (4 parameters).

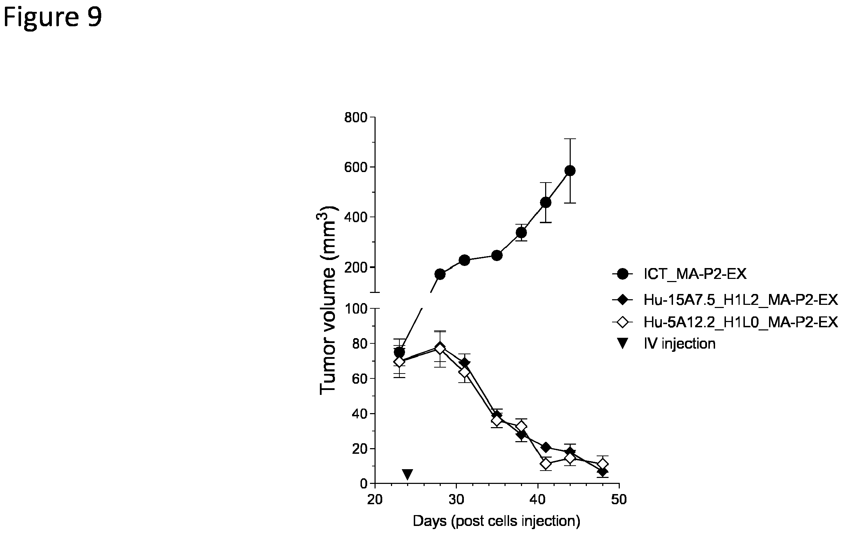

**Fig. 9: Treatment of SUM190PT grafted NSG mice with Hu-15A7.5_H1L2-Exatecan or Hu-5A12.2_H1L0-Eatecan ADC induce tumor regression period.**

NSG mice (n=5/group) were orthotopically xenografted bilaterally with the SUM190PT cells embedded in Matrigel. Three different ADC were tested: Isotypic Control, ICT-Exatecan (black circles), Hu-5A12.2_H1L0-Exatecan (open diamonds) and Hu-15A7.5_H1L2-Exatecan (black diamonds). Treatment of mice (single intra-venous injection 10 mg/kg) started when tumors reached approximately 70 mm³. Tumor sizes (n=10/group) were monitored with a caliper twice a week thereafter and sizes were reported with the following formula (LxlxhxPi/6).

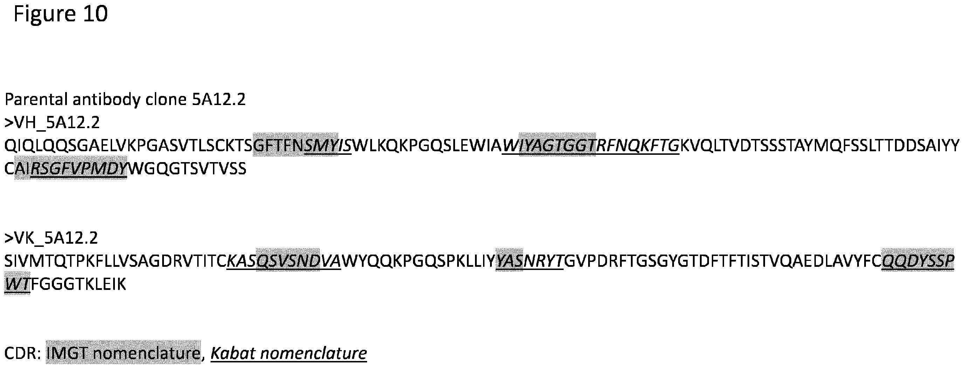

**Fig. 10: Amino acid sequence of the parental 5A12.2 monoclonal antibody**
Shown are the heavy and light chain amino acid sequences of the parental 5A12.2 monoclonal anti-Nectin-4 antibody. CDRs are identified according to IMGT and Kabat nomenclature
(IMGT nomenclature, *Kabat nomenclature*)

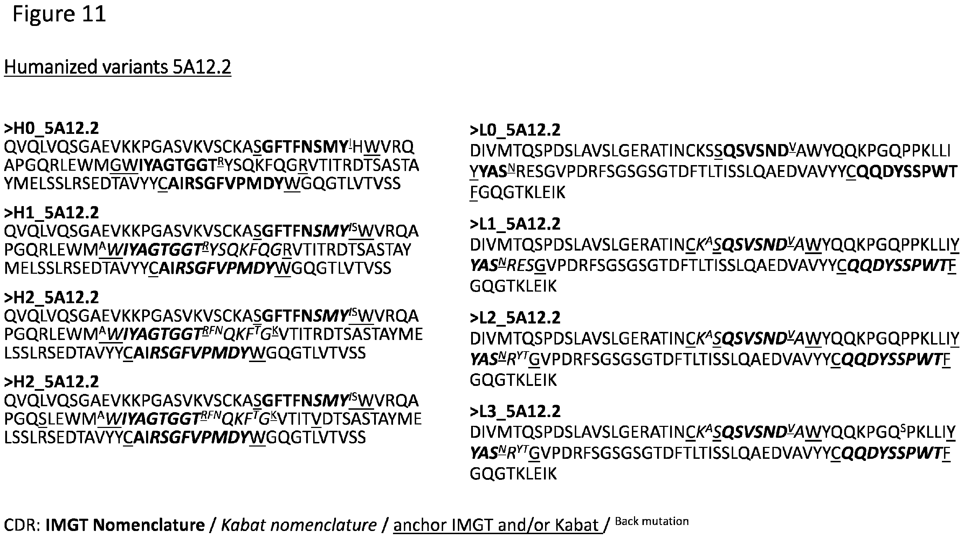

**Fig. 11: Amino acid sequence of humanized variants of the 5A12.2 monoclonal antibody**
Show are the heavy and light amino acid sequences of the humanized variants of the 5A12.2 monoclonal anti-Nectin-4 antibody. CDRs are identified according to IMGT and Kabat nomenclature. Back mutations are also identified.
(**IMGT Nomenclature** / *Kabat nomenclature* / anchor IMGT and/or Kabat / Back mutation)

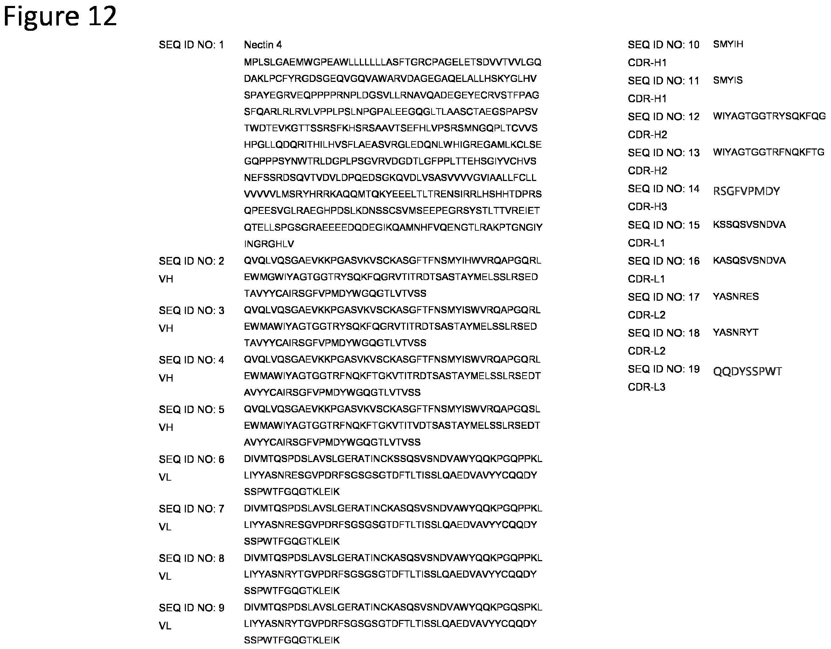

**Fig. 12: Summarized sequences of the inventive humanized antibodies**

**Examples**

Principle of epitope mapping using DMS (Deep Mutational Scanning)

DMS is a mutagenesis method that aims to perform all possible mono-substitutions on all selected residues within a given protein sequence. The DMS library is obtained in the form of DNA coding for the protein under study. In this library, each DNA strand contains a codon that is mutated with respect to the parental sequence.
This DMS DNA library is integrated into an expression plasmid specifically designed to express recombinant proteins on the yeast surface. Yeasts are then transformed and induced to allow the expression of the mono-mutated proteins on their surface. This new library (called display library) is screened by flow cytometry using fluorescent reporters to reveal the expression of the protein (anti-tag fluorescent antibody) as well as the binding of the protein to its partner (fluorescent partner).
For epitope mapping, the ideal case is to have two antibodies with compatible epitopes that can bind together on the same antigen. In this way, each of the two antibodies acts as a conformational control of the mutated antigen for the other antibody. Indeed, mono-substitutions made on the antigen can have 4 types of effects: (i) loss of affinity for the first antibody, while retaining binding for the second: this is a mutation made within the epitope of the first antibody; (ii) loss of affinity for the second antibody, while retaining binding for the first: this is a mutation in the epitope of the second antibody; (iii) loss of affinity for both antibodies: this is a so-called "destructuring" mutation which affects the conformation of the antigen and thus prevents the binding of both antibodies; (iv) no effect, the mutation is not present in the epitope of one of the two antibodies and does not cause a significant change in the conformation of the antigen. Following flow cytometry analysis, the yeast population that has lost affinity for the antibody of interest while retaining binding for the second antibody is sorted. The plasmids contained in this yeast population are extracted and sequenced by high-throughput sequencing. Analysis of the sequencing data allows the identification of mutations that have affected the binding of the antibody to its target. Thus, this analysis allows to identify the important positions on the antigen for the binding of the antibody of interest: i.e. its epitope.

Construction of the expression plasmid of the antigen

The gene corresponding to the antigen sequence Nectin-4 (32-349) was synthesized and cloned into a plasmid allowing expression on the surface of galactose-inducible yeast. In this construct, the expressed antigen carries a C-terminal HA tag. Expression plasmids are then transformed into the yeast strain *S. cerevisiae* EBY100. Two DMS libraries were generated by PCR, concerning amino acid positions 32 to 91 for Library 1 and amino acid positions 92 to 151 for Library 2. The libraries correspond to IgV domain of Nectin-4, which is known to be the target of the tested antibodies. Each mutated position carries a degenerated NNS or NNK codon encoding 20 amino acids/32 codons. Each library contained approximately 1,200 single amino acid mutants and 2,000 DNA codon mutants. The two libraries were transformed into YSD. Unsorted yeasts from the two generated libraries were sequenced to verify the efficiency of mutagenesis. One hundred percent of the expected single mutants were sequenced for library 1 and 99.9% for library 2.

Epitope compatibility

To check if the tested antibodies were compatible for epitope mapping on Nectin-4 IgV domain, i.e. that the antibodies can bind together on Nectin-4 IgV domain, antibodies were first biotinylated (EZ-Link™ Sulfo-NHS-LC-Biotin). Yeasts were induced to express Nectin-4 32-349 construct and $10^5$ yeasts were washed twice with PBS, BSA 0.1%. Yeast cells were then incubated 30 minutes with 100 nM of unlabeled antibody 1 or left as is for positive control. After 30 minutes, 500 pM of antibody 2 was added for another 30 minutes incubation. Yeasts were then incubated on ice for 15 minutes with respective fluorescent reporters, anti-HA APC for Nectin-4 expression detection and Streptavidin-PE for antibody 2 detection. After washing in PBS-BSA, resuspended yeasts were analyzed by flow cytometry. Figure 1 shows that 5A12.2_H1L0 and HA22 cannot bind simultaneously to Nectin-4 IgV domain. In contrast, 15A7.5_H1L2 mAb can bind Nectin-4 IgV domain simultaneously with either 5A12.2_H1L0 or HA22 (Enfortumab).

Induction of antigen expression and FACS-sorting of libraries

Yeasts expressing Nectin4 mono-mutants were induced for protein expression. Induction of transformed yeast was done in SG-CAA induction medium [6.7 g/L yeast nitrogen base without casamino acids, 20 g/L glucose, 5 g/L casamino acids, 100 mM sodium phosphate, pH 6.0]. For cytometric analysis/sorting, between $10^6$ and $10^8$ induced cells were washed with 1 mL of PBSF (PBS, 0.1% BSA). The cells were then resuspended in an appropriate volume of solution containing 100 pM of the biotinylated HA22 antibody and Dye650-labeled 15A7.5_H1L2. After 1h to 3h of incubation at 20°C with agitation, the cells were washed with 1 mL of ice-cold PBSF and then incubated with the streptavidin on ice for 15 min. The cells were then analyzed/sorted on a BD FACSAria™ III cytometer using the BD FACSdiva™ Diva software. As shown in Figure 2, the single-stained yeasts in each experiment were sorted and plasmids from each sorted yeast population were extracted and prepared for sequencing. A two-step PCR was performed: a first step to amplify the region of interest and a second step to add the Illumina adapters needed for sequencing. Sequencing was performed on an Illumina iSeq100 instrument ($2 \times 150$ bp, 300 cycles) with at least 150,000 reads per population. The data were then processed through an analysis pipeline using dedicated proprietary scripts. Poor quality sequences (Q<30) were removed, then mono-mutants were detected and counted. For each mono-mutation, the frequency measured in the unsorted population was compared to the frequency of the mutant in the sorted population. An enrichment value for the mutant was then calculated according to the following formula:

$$Enrichment\ mutant = \frac{Frequency\ mutant,\ sorted\ population}{Frequency\ mutant, unsorted\ population}$$

A high enrichment value indicates that the mutation under consideration caused a loss of recognition of the antibody for its antigen, while maintaining recognition of the other antibody (conformational control).

Data interpretation

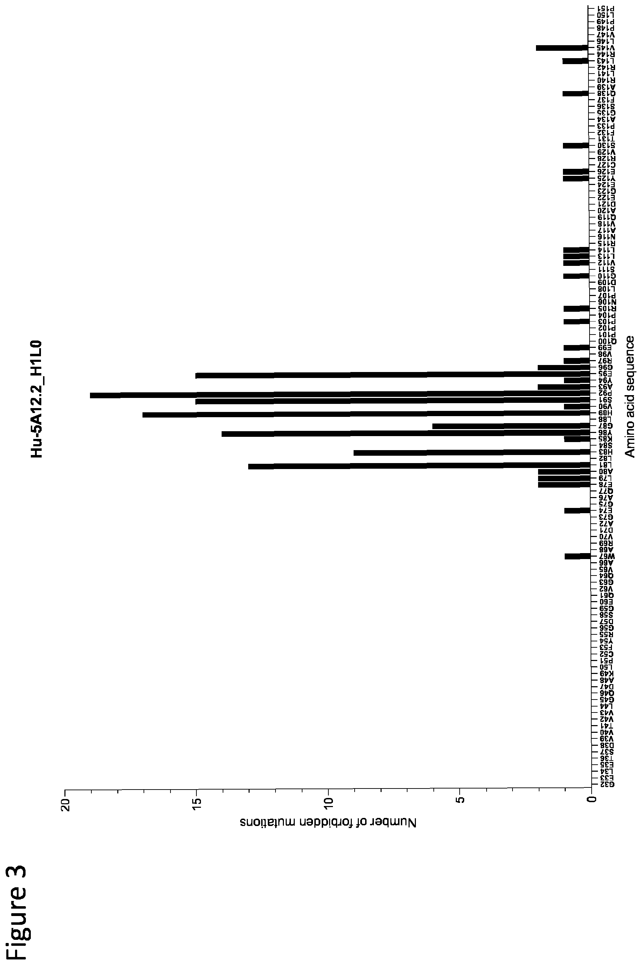

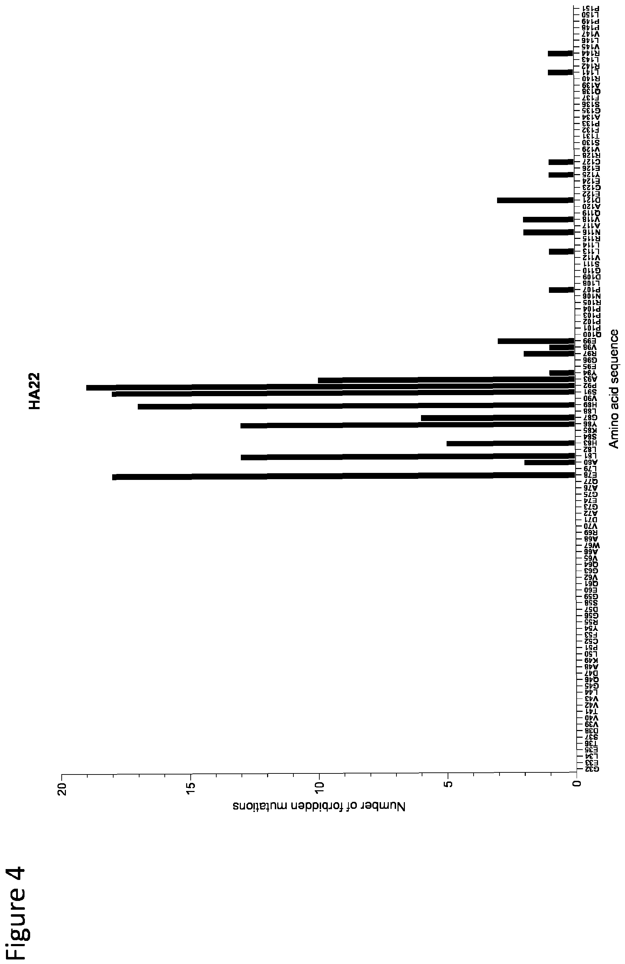

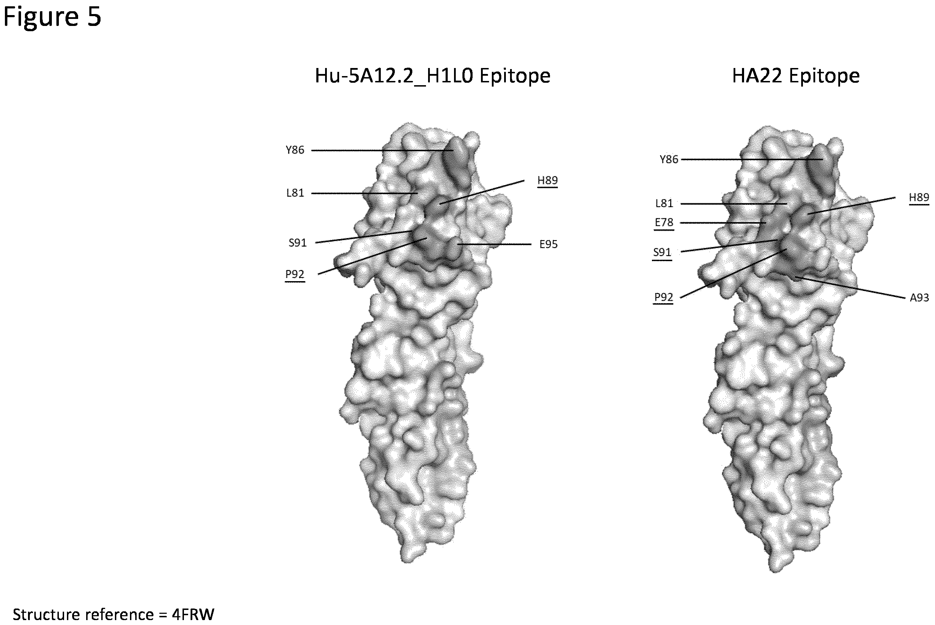

The result of the processing of the NGS sequencing data is represented as a bar graph that aggregates the enrichment values for all single mutants present in the DMS libraries (Figures 3, 4). Between 16 and 19 forbidden mutations indicate high impact and positions classified in this category are the most likely to be indirect interaction with the considered IgG. Between 10 and 15 forbidden mutations indicate medium impact positions, and between 5 and 9 forbidden mutations indicate low impact positions. Figure 5 shows an analysis of the structure of Nectin-4 (4FRW, pdb) to highlight the important and most important epitope residues for each of the antibody tested. Table 1 lists these residues.

Table 1: Important and most important residues of the Nectin-4 epitopes of monoclonal antibodies HA22 and 5A12.2_H1L0

| Antibody | Epitope residues | Most important epitope residues |
|---|---|---|
| HA22 | E78, L81, H83, Y86, G87, H89, S91, P92 and A93 | E78, H89, S91 and P92 |
| 5A12.2_H1L0 | L81, H83, Y86, G87, H89, S91, P92 and E95 | H89 and P92 |

Humanization of chimeric antibodies

The methodology used is the "CDR grafting": the 3 mouse CDR (regions determining the specificity of the antibody) of each antibody chain (heavy, VH or light, VL) are grafted on the closest human germline backbone. This methodology takes also in consideration the back-mutations of essential amino-acids (anchors of the CDR, Vernier's residues, interface VH/VL, etc....) in the FrameWork regions (FR).

Chimeric and humanized antibodies generation, production, purification and control

Light chain expression vector is coding for a V kappa chain. Depending on the payload used, 2 different heavy chain expression vectors were used, one coding for a Fc fragment with D265C (ThiomAb) L234A and L235A mutations, one coding for a Fc fragment with P331S, L234F and L235E mutations. Both Fc fragments are "Fc-silent". The sequences of anti-Nectin-4 Enfortumab (HA22) were also cloned in the same vectors.

Light and heavy chain vectors were transfected in HEK293 seeded with a 1.2/1 ratio. After 6 days of production, culture supernatants were clarified and mAbs were purified using MabSelect PrismA resin (GE Healthcare) according to the manufacturer's instructions. 0.5M Glycine, 3M NaCl, pH8.9 was used as binding buffer, and 0.1 M Citrate pH 3 was used for elution. Instant neutralization was done with 10% (V/V) 1M Tris-HCl pH 9. Monoclonal antibodies were then dialyzed against PBS 1X pH 7.4 (Mini dialysis devices, 2 mL-10k, Thermo Scientific) followed by filtration on 0.22 $\mu$M filter (Milelex GV hydro-philic PVDF, Millipore). Concentration was determined with a Nanodrop 2000 Spectrophotometer (Thermo Scientific) considering the specific extinction coefficient (E1%280nm) of each monoclonal antibody. Purity was determined by UPLC-SEC using an Acquity UPLC-HClass Bio (Waters) using a Protein-BEH 200A column equilibrated in 0.2 M NaPO4, 0.3 M NaCl pH 6.9 supplemented with 10% isopropanol. The mass of the antibodies was determined in a Xevo G2-S Q-Tof mass spectrophotometer (Waters) using a reversed-phase column (PLRP-S 4000A, Agilent technologies). All samples were analyzed after deglycosylation with PNGase F glycosidase (New England Biolabs) at 37°C, according to the manufacturer's instructions. Fragmentation and/or aggregation of the final material was evaluated by SDS-PAGE. Endotoxin load was determined using a chromogenic LAL-kinetic assay (Charles River Endosafe).

Table 2 reports production yields at small scale (30 mL) of the parental chimeric 5A12.2 and all the humanized variants.

Table 3 reports production yields at medium scale (0.5-1.4 L) of the parental chimeric 5A12.2 and humanized variants 5A12.2_H1L0 and 5A12.2_H1L1.

Table 2: Small scale production yield of humanized variant versus that of parental chimeric 5A12.2 antibody. CHt: chimeric antibody, HUt: humanized antibody

| Antibody | Amount produced (mg) | Volume (mL) | Yield (mg/mL) |
|---|---|---|---|
| CHt_5A12.2 | 2.21 | 30 | 0.074 |
| HUt_5A12.2_H0L0 | 5.94 | 30 | 0.198 |
| HUt_5A12.2_H0L1 | 6.1 | 30 | 0.203 |
| HUt_5A12.2_H0L2 | 5.77 | 30 | 0.192 |
| HUt_5A12.2_H0L3 | 6.07 | 30 | 0.202 |
| HUt_5A12.2_H1L0 | 5.41 | 30 | 0.180 |
| HUt_5A12.2_H1L1 | 5.15 | 30 | 0.172 |
| HUt_5A12.2_H1L2 | 6.08 | 30 | 0.203 |
| HUt_5A12.2_H1L3 | 5.60 | 30 | 0.187 |
| HUt_5A12.2_H2L0 | 4.44 | 30 | 0.148 |
| HUt_5A12.2_H2L1 | 4.73 | 30 | 0.158 |
| HUt_5A12.2_H2L2 | 5.73 | 30 | 0.191 |
| HUt_5A12.2_H2L3 | 3.54 | 30 | 0.118 |
| HUt_5A12.2_H3L0 | 5.15 | 30 | 0.172 |
| HUt_5A12.2_H3L1 | 5.95 | 30 | 0.198 |
| HUt_5A12.2_H3L2 | 5.74 | 30 | 0.191 |
| HUt_5A12.2_H3L3 | 6.07 | 30 | 0.202 |

Table 3: Medium scale production yield of humanized variant versus that of parental chimeric 5A12.2 antibody. CHt: chimeric antibody, HUt: humanized antibody

| Antibody | Amount produced (mg) | Volume (mL) | Yield (mg/mL) |
|---|---|---|---|
| CHt_5A12.2 | 129.5 | 1400 | 0.093 |
| HUt_5A12.2_H1L0 | 76.5 | 500 | 0.153 |
| HUt_5A12.2_H1L1 | 83.1 | 500 | 0.166 |

Antibody conjugation

A cysteine reactive linker-amanitin compound from Heidelberg Pharma with a cleavable linker (valine alanine) to engineered cysteine residues of selected anti-Nectin-4 ThiomAb (D265C, L234A, L235A) antibodies using maleimide chemistry. Briefly, ThiomAb antibodies in PBS 1X pH 7.4 were reduced with TCEP and interchains disulfides were re-oxidized by dehydroascorbic acid. Subsequently, the engineered cysteines were used for conjugation with cysteine reactive linker amanitin compound. The conjugates were purified by dialysis. The drug-antibody ratio (DAR) according to LC-MS analysis was comprised between 1.44 and 1.79 toxins per conjugated mAb. As determined by SEC-HPLC, less than 2% material was aggregated.

The cysteine reactive linker-exatecan compound Maleimide-Propio-PEG2-PSAR10-glucuronide-exatecan (MabLink) was conjugated to cysteine residues of selected anti-Nectin-4 mAbs (P331S, L234F and L235E). In brief, mAbs in PBS 1X, 1 mM EDTA were reduced with 14 molar equivalent of TCEP for 2 hours at 37°C, after which the buffer was exchanged (Amicon ultra 30 kDa) to 100 mM $KPO_4$, 1 mM EDTA pH 7.4. Twelve molar equivalents of the cysteine reactive linker-exatecan compound were used for conjugation with reactive cysteines for 35 min at room temperature. The final exchange buffer was performed in 20 mM His pH 6.0 before filtration 0.22 $\mu$M filter. The drug-antibody ratio (DAR) according to LC-MS analysis was comprised between 7.77 and 7.82 toxins per conjugated mAb. As determined by SEC-HPLC, less than 8% material was aggregated.

Cell lines

Human triple negative breast cancer cell line SUM190PT (BioIVT, Westbury, NY) was cultured in Ham's F12 medium with 5% fetal bovine serum, 1% non-essential amino acids, 1% Hepes, 1% insulin, 1 $\mu$g/mL hydrocortisone, 6.8 ng/mL Triiodo L-tyrosine, 100 IU/mL penicillin, 100 $\mu$g/mL streptomycin and 2 mM glutamine. Human breast carcinoma T47D cell line was cultured in RPMI supplemented with 10% fetal bovine serum, 100 IU/mL penicillin, 100 $\mu$g/mL streptomycin. The Human colorectal carcinoma Nectin-4 transfectant (HCT116-N4) was cultured in RPMI supplemented with 10% fetal bovine serum, 2 mM L-Glutamine, 1X MEM Non-essential amino acids and 20 $\mu$g/mL Hygromycin B.

*In vitro* cytotoxicity assay

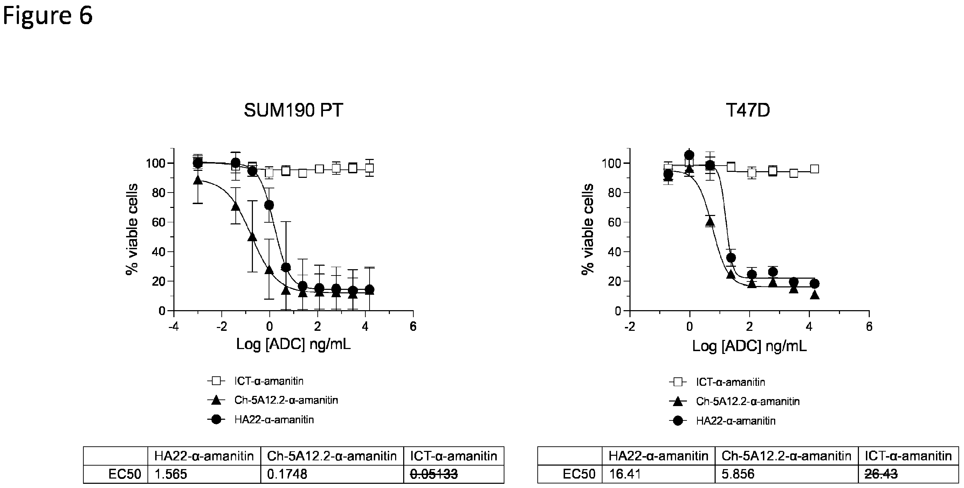

To analyze the *in vitro* cytotoxic activity of $\alpha$-amanitin ADC and Exatecan ADC, cell viability was assessed using the AlamarBlue staining protocol as recommended by the manufacturer (Biosource, CA, USA). The test incorporates a fluorescent oxidation-reduction indicator. Fluorescence intensity is proportional to cellular metabolic reduction. Experiments were done by incubating 3,000 cells/well (SUM190PT, T47D or HCT116-Nectin-4) in triplicate with serial dilutions of ADC at Day 0 in 96-wells plates. AlamarBlue was measured at Day 5 by incubating 1/10 volume of AlamarBlue solution for 2 h at 37° C and read at 595 nm (FLUOstar Optima, BMG Labtech). Figure 6 shows that the parental 5A12.2 anti-Nectin-4 monoclonal antibody has a lower $EC_{50}$ than HA22. Figure 8 shows that humanized variants H1L0 and H1L1 of monoclonal antibody 5A12.2 display similar EC50 in comparison to another humanized anti-Nectin-4 antibody.

Flow Cytometry

T47D cells (50,000) naturally expressing Nectin-4 were incubated with dose range of the indicated antibodies. After washing, cells were then stained with phycoerythrine-conjugated goat anti human antibody (5 $\mu$g/mL) Jackson Immuno Research). After fixation, cells were stained with a viability dye (e780, Invitrogen) before flow cytometry acquisition. Figure 7 shows that all 5A12.2 humanized variants except H0 variants have retained similar apparent affinity in comparison to the parental 5A12.2 monoclonal antibody.

Mouse experiments

NOD/SCID (nonobese diabetic/severe combined immunodeficient)/gc null mice (NSG) were obtained from Charles River Laboratory (Margate, UK). Six to seven-weeks old females (n=5/group) were orthotopically xenografted bilaterally with the SUM190PT ($0.5 \times 10^6$) cells embedded in Matrigel. Treatment with ADC was performed as mentioned in the respective experiments. Tumor sizes (n=10/group) were monitored with a caliper twice a week thereafter and sizes were reported with the following formula (LxlxhxPi/6). Figure 9 shows that, in vivo, a single injection of humanized variant 5A12.2_H1L0 monoclonal antibody coupled to Exatecan can induce regression of triple negative breast cancer cell line SUM190PT.

Determination of antibody affinities on octet platform

Affinities of the various monoclonal antibodies were determined on the Octet Red96 platform which is a system based on bio-layer interferometry (BLI) technology. Two types of analytes were used in these studies: (*i*) a his-tagged recombinant extra cellular domain of the Nectin-4 protein (R&D systems/Biotechne); (*ii*) a homodimer recombinant Fc protein, corresponding to the IgV domain of human Nectin-4 fused to human Fc domain. An AHC sensor (anti human Fc) was used for the recombinant his-tagged Nectin-4 EC analyte and a FAB2G sensor was used for the Fc fusion recombinant IgV domain of Nectin-4. The ligand of interest diluted in 1X kinetic buffer is loaded on the selected sensor. The analyte remains in solution at working concentrations in 1x kinetic buffer ranging from 600 nM to 1.56 nM. Each run is performed at +26°C under 1,000 rpm shaking and is made of 5 steps; loading step, equilibration step, association step, dissociation step and regeneration. For the analysis of generated data, the standard 1:1 Langmuir model was chosen for the AHC sensor and the 1:2 bivalent analyte model was chosen for the FAB2G sensor. Both analysis models are run by the Octet software. Table 2 indicates that the affinity for His-tagged recombinant Nectin-4 is similar for 5A12.2 and HA22 monoclonal antibodies. In contrast, the affinity for the Fc fusion IgV Nectin-4 domain is markedly different between 5A12.2 and HA22 suggesting that 5A12.2 has a better avidity for its dimeric epitope on Nectin-4 than HA22.

Table 4: $K_D$ on 5A12.2 and HA22 monoclonal antibodies

| Antibody | $K_D$ for His-Nectin-4 | $K_D$ for Fc-Nectin-4 |
|----------|------------------------|------------------------|
| Ch-5A12.2 | 4.2 nM | 5.3 nM |
| HA22 | 5.2 nM | 12.9 nM |

Table 5 reports the affinity determination for the various humanization variants of monoclonal antibody 5A12.2. Only the recombinant his-tagged Nectin-4 EC analyte was used here. The affinity of the various humanized variant is similar to that the parental antibody.

Table 5: Affinity determination of various humanized variants of 5A12.2 in comparison to the parental antibody

| Antibody | $K_D$ (nM) | Ka (1/Ms) | Kdis (1/s) | $R^2$ |
|----------|-----------|-----------|------------|-------|
| Ch-5A12.2 | 3.4 | 2.75E+05 | 9.28E-04 | 0.99 |
| Hu-5A12.2_H1L0 | 4.6 | 2.66E+05 | 1.23E-03 | 0.98 |
| Hu-5A12.2_H1L1 | 4.7 | 2.67E+05 | 1.24E-03 | 0.98 |
| Hu-5A12.2_H2L0 | 4.4 | 3.6E+05 | 1.6E-03 | 0.96 |
| Hu-5A12.2_H2L1 | 4.2 | 3.89E+05 | 1.64E-03 | 0.95 |
| Hu-5A12.2_H3L0 | 4.3 | 3.4E+05 | 1.45E-03 | 0.96 |
| Hu-5A12.2_H3L1 | 4.2 | 3.49E+05 | 1.46E-03 | 0.96 |

**Claims**

1. A humanized monoclonal antibody or antigen-binding fragment thereof, which binds to a discontinuous epitope on Nectin-4 consisting of amino acids L81, H83, Y86, G87, H89, S91, P92 and E95 of SEQ ID NO: 1 as determined using Deep Mutational Scanning (DMS).

2. The humanized antibody according to claim 1, comprising

   (a) a variable heavy chain (VH) region comprising an amino acid sequence according to any one of SEQ ID NOs: 2-5, or an amino acid sequence having an identity thereto of at least 85%, at least 90%, at least 95% or at least 99%,

and including complementarity-determining regions (CDRs) CDR-H1 according to SEQ ID NO: 10 or 11, CDR-H2 according to SEQ ID NO: 12 or 13 and CDR-H3 according to SEQ ID NO: 14,

(b) a variable light chain (VL) region comprising an amino acid sequence according to any one of SEQ ID NOs: 6-9, or an amino acid sequence having an identity thereto of at least 85%, at least 90%, at least 95% or at least 99%,

and including CDR-L1 according to SEQ ID NO: 15 or 16, CDR-L2 according to SEQ ID NO: 17 or 18 and CDR-L3 according to SEQ ID NO: 19

3. The humanized antibody according to claim 2, comprising

(i) a VH region comprising an amino acid sequence according to SEQ ID NO: 2 or an amino sequence having an identity thereof of at least 85%, at least 90%, at least 95% or at least 99%, and
a VL region comprising an amino acid sequence according to SEQ ID NO: 6 or an amino sequence having an identity thereof of at least 85%, at least 90%, at least 95% or at least 99%,
(ii) a VH region comprising an amino acid sequence according to SEQ ID NO: 3, or an amino sequence having an identity thereof of at least 85%, at least 90%, at least 95% or at least 99%, and
a VL region comprising an amino acid sequence according to SEQ ID NO: 7 or an amino sequence having an identity thereof of at least 85%, at least 90%, at least 95% or at least 99%,
(iii) a VH region comprising an amino acid sequence according to SEQ ID NO:4 or an amino sequence having an identity thereof of at least 85%, at least 90%, at least 95% or at least 99%, and
a VL region comprising an amino acid sequence according to SEQ ID NO: 8, or an amino sequence having an identity thereof of at least 85%, at least 90%, at least 95% or at least 99%,
(iv) a VH region comprising an amino acid sequence according to SEQ ID NO: 5, or an amino sequence having an identity thereof of at least 85%, at least 90%, at least 95% or at least 99%, and

a VL region comprising an amino acid sequence according to SEQ ID NO: 9, or an amino sequence having an identity thereof of at least 85%, at least 90%, at least 95% or at least 99%.

4. The humanized antibody according to claim 2 or 3, comprising

(a) a VH region comprising an amino acid sequence according to SEQ ID NO:2-5, and
(b) a VL region comprising an amino acid sequence according to SEQ ID NO: 6-9.

5. The humanized antibody or antigen-binding fragment of any one of claims 1-4, which is a multispecific antibody, in particular a bispecific antibody, such as a biparatopic antibody or an antigen-binding fragment thereof.

6. The humanized antibody or antigen-binding fragment of any one of claims 1-5, which is an antibody of class IgG, e.g. of subclass IgG1, IgG2, IgG3 of IgG4, of class IgM, of class IgA or an antigen-binding fragment thereof, or which is a single-chain antibody, or an antibody Fv fragment.

7. The humanized antibody or antigen-binding fragment of any of claims 1 to 6, comprising a labeling group and/or an effector group being coupled to the antibody or antigen-binding fragment.

8. The humanized antibody or antigen-binding fragment of claim 7, wherein the labeling group is a dye, a paramagnetic, radioactive or fluorogenic group that is detectable upon imaging.

9. The humanized antibody or antigen-binding fragment of claim 8 wherein the effector group is a therapeutic group, in particular a cytotoxic agent.

10. An antibody-drug conjugate, comprising

a humanized antibody or antigen-binding fragment thereof according to any one of claims 1-9, and
a drug conjugated to a reactive amino acid residue on the antibody or antigen-binding fragment, e.g., an amino acid residue having a side chain comprising an amino, hydroxy or thiol group, or a reactive group in the antibody glycan structure.

11. The antibody-drug conjugate of claim 10, wherein the drug is conjugated to a reactive thiol group in the side chain of a cysteine residue on the antibody or antigen-binding fragment thereof.

12. The antibody-drug conjugate of claim 10 or 11, wherein the drug is conjugated to the antibody or antigen-binding fragment thereof via a linker, particularly wherein the linker is a cleavable linker, such as a linker subject to cleavage by a glucuronidase, and/or wherein the linker is a peptidic linker.

13. The humanized antibody or fragment of any one of claims 1-10 for use in medicine, particularly for therapeutic or diagnostic applications including in vitro and in vivo diagnostic applications.

14. The humanized antibody or fragment for use according to claim 13 in a method of preventing and/or treating cancer and/or inflammatory disorders.

15. The humanized antibody or fragment for use according to claim 13 or 14, wherein the cancer is associated with Nectin-4 overexpression.

16. The humanized antibody or fragment for use according to any one of 13-15, wherein the cancer is urothelial, endometrial, cervical, colorectal, liver, thyroid, breast, pancreatic, lung, ovarian head and neck and/or esophagus cancer.

17. A nucleic acid coding for one or more of the antibodies according to any one claims 1-9, or for at least one VL and/or one VH of any one of the antibodies of claims 1-9.

18. A vector comprising the nucleic acid according to claim 17.

19. A host cell comprising the vector of claim 18.

20. A pharmaceutical composition comprising a humanized antibody or antigen-binding fragment thereof according to any of claims 1-9, an antibody-drug conjugate according to any one of claims 10-12, or a vector according to claim 18, and optionally one or more pharmaceutical excipients.

Figure 1

EP 4 324 849 A1

Figure 2

# Figure 3

Hu-5A12.2_H1L0

Number of forbidden mutations

Amino acid sequence

Figure 4

HA22

Number of forbidden mutations

Amino acid sequence

# Figure 5

Hu-5A12.2_H1L0 Epitope

HA22 Epitope

Structure reference = 4FRW

## Figure 6

**SUM190 PT**

|      | HA22-α-amanitin | Ch-5A12.2-α-amanitin | ICT-α-amanitin |
|------|-----------------|----------------------|----------------|
| EC50 | 1.565           | 0.1748               | ~~0.05133~~    |

**T47D**

|      | HA22-α-amanitin | Ch-5A12.2-α-amanitin | ICT-α-amanitin |
|------|-----------------|----------------------|----------------|
| EC50 | 16.41           | 5.856                | ~~26.43~~      |

EP 4 324 849 A1

Figure 7

H0 variants

H1 variants

H2 variants

H3 variants

Figure 8

| | EC50 |
|---|---|
| Hu-15A7.5_H1L2_MA-P2-EX | 0.04927 |
| ICT_MA-P2-EX | ~ 483986384 |
| Hu-5A12.2_H1L0_MA-P2-EX | 0.07430 |
| Hu-5A12.2_H1L1_MA-P2-EX | 0.02082 |

EP 4 324 849 A1

# Figure 9

# Figure 10

Parental antibody clone 5A12.2

>VH_5A12.2

QIQLQQSGAELVKPGASVTLSCKTSGFTFNSMYISWLKQKPGQSLEWIAWIYAGTGGTRFNQKFTGKVQLTVDTSSSTAYMQFSSLTTDDSAIYY
CAIRSGFVPMDYWGQGTSVTVSS

>VK_5A12.2

SIVMTQTPKFLLVSAGDRVTITCKASQSVSNDVAWYQQKPGQSPKLLIYYASNRYTGVPDRFTGSGYGTDFTFTISTVQAEDLAVYFCQQDYSSP
WTFGGGTKLEIK

CDR: IMGT nomenclature, *Kabat nomenclature*

EP 4 324 849 A1

# Figure 11

## Humanized variants 5A12.2

**>H0_5A12.2**
QVQLVQSGAEVKKPGASVKVSCKA**S**GFTFNSMY<sup>I</sup>H<u>W</u>VRQ
APGQRLEWM<u>GWIYAGTGGT</u><sup>R</sup>YSQKFQG<u>R</u>VTITRDTSASTA
YMELSSLRSEDTAVYY<u>C</u>**AIRSGFVPMDY**<u>W</u>GQGTLVTVSS

**>H1_5A12.2**
QVQLVQSGAEVKKPGASVKVSCKA<u>S</u>GFT**FNSMY**<sup>IS</sup><u>W</u>VRQA
PGQRLEWM<sup>A</sup>*WIYAGTGGT*<sup>R</sup>*YSQKFQG*<u>R</u>VTITRD*T*SASTAY
MELSSLRSEDTAVYY<u>C</u>*AIRSGFVPMDY*<u>W</u>GQGTLVTVSS

**>H2_5A12.2**
QVQLVQSGAEVKKPGASVKVSCKA<u>S</u>GFT**FNSMY**<sup>IS</sup><u>W</u>VRQA
PGQRLEWM<sup>A</sup>*WIYAGTGGT*<sup>RFN</sup>*QKF*<sup>T</sup>*G*<sup>K</sup>VTITRDTSASTAYME
LSSLRSEDTAVYY<u>C</u>*AIRSGFVPMDY*<u>W</u>GQGTLVTVSS

**>H2_5A12.2**
QVQLVQSGAEVKKPGASVKVSCKA<u>S</u>GFT**FNSMY**<sup>IS</sup><u>W</u>VRQA
PGQ**S**LEWM<sup>A</sup>*WIYAGTGGT*<sup>RFN</sup>*QKF*<sup>T</sup>*G*<sup>K</sup>VTIT**V**DTSASTAYME
LSSLRSEDTAVYY<u>C</u>*AIRSGFVPMDY*<u>W</u>GQGTLVTVSS

**>L0_5A12.2**
DIVMTQSPDSLAVSLGERATINCKSS**QSVSND**<sup>V</sup>A<u>W</u>YQQKPGQPPKLLI
<u>Y</u>YAS<sup>N</sup>RESGVPDRFSGSGSGTDFTLTISSLQAEDVAVYY<u>C</u>**QQDYSSPWT**
<u>F</u>GQGTKLEIK

**>L1_5A12.2**
DIVMTQSPDSLAVSLGERATIN<u>C</u>*K*<sup>A</sup><u>S</u>**QSVSND**<sup>V</sup>A<u>W</u>YQQKPGQPPKLLI<u>Y</u>
*YAS*<sup>N</sup>*RES*<u>G</u>VPDRFSGSGSGTDFTLTISSLQAEDVAVYY<u>C</u>*QQDYSSPWT*<u>F</u>
GQGTKLEIK

**>L2_5A12.2**
DIVMTQSPDSLAVSLGERATIN<u>C</u>*K*<sup>A</sup><u>S</u>**QSVSND**<sup>V</sup>A<u>W</u>YQQKPGQPPKLLI<u>Y</u>
*YAS*<sup>N</sup>*R*<sup>YT</sup><u>G</u>VPDRFSGSGSGTDFTLTISSLQAEDVAVYY<u>C</u>*QQDYSSPWT*<u>F</u>
GQGTKLEIK

**>L3_5A12.2**
DIVMTQSPDSLAVSLGERATIN<u>C</u>*K*<sup>A</sup><u>S</u>**QSVSND**<sup>V</sup>A<u>W</u>YQQKPGQ<sup>S</sup>PKLLI<u>Y</u>
*YAS*<sup>N</sup>*R*<sup>YT</sup><u>G</u>VPDRFSGSGSGTDFTLTISSLQAEDVAVYY<u>C</u>*QQDYSSPWT*<u>F</u>
GQGTKLEIK

CDR: **IMGT Nomenclature** / *Kabat nomenclature* / <u>anchor IMGT and/or Kabat</u> / <sup>Back mutation</sup>

EP 4 324 849 A1

# Figure 12

SEQ ID NO: 1  Nectin 4

MPLSLGAEMWGPEAWLLLLLLLASFTGRCPAGELETSDVVTVVLGQ
DAKLPCFYRGDSGEQVGQVAWARVDAGEGAQELALLHSKYGLHV
SPAYEGRVEQPPPPRNPLDGSVLLRNAVQADEGEYECRVSTFPAG
SFQARLRLRVLVPPLPSLNPGPALEEGQGLTLAASCTAEGSPAPSV
TWDTEVKGTTSSRSFKHSRSAAVTSEFHLVPSRSMNGQPLTCVVS
HPGLLQDQRITHILHVSFLAEASVRGLEDQNLWHIGREGAMLKCLSE
GQPPPSYNWTRLDGPLPSGVRVDGDTLGFPPLTTEHSGIYVCHVS
NEFSSRDSQVTVDVLDPQEDSGKQVDLVSASVVVVGVIAALLFCLL
VVVVVLMSRYHRRKAQQMTQKYEEELTLTRENSIRRLHSHHTDPRS
QPEESVGLRAEGHPDSLKDNSSCSVMSEEPEGRSYSTLTTVREIET
QTELLSPGSGRAEEEEDQDEGIKQAMNHFVQENGTLRAKPTGNGIY
INGRGHLV

SEQ ID NO: 2  VH

QVQLVQSGAEVKKPGASVKVSCKASGFTFNSMYIHWVRQAPGQRL
EWMGWIYAGTGGTRYSQKFQGRVTITRDTSASTAYMELSSLRSED
TAVYYCAIRSGFVPMDYWGQGTLVTVSS

SEQ ID NO: 3  VH

QVQLVQSGAEVKKPGASVKVSCKASGFTFNSMYISWVRQAPGQRL
EWMAWIYAGTGGTRYSQKFQGRVTITRDTSASTAYMELSSLRSED
TAVYYCAIRSGFVPMDYWGQGTLVTVSS

SEQ ID NO: 4  VH

QVQLVQSGAEVKKPGASVKVSCKASGFTFNSMYISWVRQAPGQRL
EWMAWIYAGTGGTRFNQKFTGKVTITRDTSASTAYMELSSLRSEDT
AVYYCAIRSGFVPMDYWGQGTLVTVSS

SEQ ID NO: 5  VH

QVQLVQSGAEVKKPGASVKVSCKASGFTFNSMYISWVRQAPGQSL
EWMAWIYAGTGGTRFNQKFTGKVTITVDTSASTAYMELSSLRSEDT
AVYYCAIRSGFVPMDYWGQGTLVTVSS

SEQ ID NO: 6  VL

DIVMTQSPDSLAVSLGERATINCKSSQSVSNDVAWYQQKPGQPPKL
LIYYASNRESGVPDRFSGSGSGTDFTLTISSLQAEDVAVYYCQQDY
SSPWTFGQGTKLEIK

SEQ ID NO: 7  VL

DIVMTQSPDSLAVSLGERATINCKASQSVSNDVAWYQQKPGQPPKL
LIYYASNRESGVPDRFSGSGSGTDFTLTISSLQAEDVAVYYCQQDY
SSPWTFGQGTKLEIK

SEQ ID NO: 8  VL

DIVMTQSPDSLAVSLGERATINCKASQSVSNDVAWYQQKPGQPPKL
LIYYASNRYTGVPDRFSGSGSGTDFTLTISSLQAEDVAVYYCQQDY
SSPWTFGQGTKLEIK

SEQ ID NO: 9  VL

DIVMTQSPDSLAVSLGERATINCKASQSVSNDVAWYQQKPGQSPKL
LIYYASNRYTGVPDRFSGSGSGTDFTLTISSLQAEDVAVYYCQQDY
SSPWTFGQGTKLEIK

SEQ ID NO: 10  SMYIH
CDR-H1

SEQ ID NO: 11  SMYIS
CDR-H1

SEQ ID NO: 12  WIYAGTGGTRYSQKFQG
CDR-H2

SEQ ID NO: 13  WIYAGTGGTRFNQKFTG
CDR-H2

SEQ ID NO: 14  RSGFVPMDY
CDR-H3

SEQ ID NO: 15  KSSQSVSNDVA
CDR-L1

SEQ ID NO: 16  KASQSVSNDVA
CDR-L1

SEQ ID NO: 17  YASNRES
CDR-L2

SEQ ID NO: 18  YASNRYT
CDR-L2

SEQ ID NO: 19  QQDYSSPWT
CDR-L3

Europäisches
Patentamt

European
Patent Office

Office européen
des brevets

# EUROPEAN SEARCH REPORT

Application Number

EP 22 19 1026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/069508 A1 (UNIV AIX MARSEILLE [FR]; INST NAT SANTE RECH MED [FR] ET AL.) 15 April 2021 (2021-04-15) | 1,13, 17-20 | INV. C07K16/28 |
| Y | *whole document* *p. 2, 17, 19, 20, 21, 23, 25, 26, 28, 30, 31, 34, 35* | 2-12, 14-16 | |
| Y | WU H ET AL: "Humanization of a murine monoclonal antibody by simultaneous optimization of framework and CDR residues", JOURNAL OF MOLECULAR BIOLOGY, ACADEMIC PRESS, UNITED KINGDOM, vol. 294, no. 1, 19 November 1999 (1999-11-19), pages 151-162, XP004461859, ISSN: 0022-2836, DOI: 10.1006/JMBI.1999.3141 *whole document* *p. 3-6* | 2-11, 14-16 | |
| Y | WO 2021/151984 A1 (INNATE PHARMA [FR]) 5 August 2021 (2021-08-05) | 2-12, 14-16 | TECHNICAL FIELDS SEARCHED (IPC) C07K |
| A | *whole document* *claims 1-3* *p. 38, 39* | 1,13, 17-20 | |
| A | EP 3 347 048 B1 (INST NAT SANTE RECH MED [FR]; UNIV AIX MARSEILLE [FR] ET AL.) 1 April 2020 (2020-04-01) | 1-20 | |
| A | WO 2021/213434 A1 (MABWELL SHANGHAI BIOSCIENCE CO LTD [CN]) 28 October 2021 (2021-10-28) *whole document* *pages 3-6* | 1-20 | |

The present search report has been drawn up for all claims

| Place of search | Date of completion of the search | Examiner |
|---|---|---|
| Munich | 21 December 2022 | Baumbach, Janina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 1026

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2022

| Patent document cited in search report | | Publication date | Patent family member(s) | | Publication date |
|---|---|---|---|---|---|
| WO 2021069508 | A1 | 15-04-2021 | AU | 2020364959 A1 | 07-04-2022 |
| | | | CA | 3156451 A1 | 15-04-2021 |
| | | | CN | 114514246 A | 17-05-2022 |
| | | | EP | 4041765 A1 | 17-08-2022 |
| | | | IL | 291966 A | 01-06-2022 |
| | | | JP | 2022551537 A | 09-12-2022 |
| | | | KR | 20220079614 A | 13-06-2022 |
| | | | WO | 2021069508 A1 | 15-04-2021 |
| WO 2021151984 | A1 | 05-08-2021 | AU | 2021213421 A1 | 23-06-2022 |
| | | | CA | 3160557 A1 | 05-08-2021 |
| | | | CN | 114845740 A | 02-08-2022 |
| | | | EP | 4096717 A1 | 07-12-2022 |
| | | | WO | 2021151984 A1 | 05-08-2021 |
| EP 3347048 | B1 | 01-04-2020 | EP | 3347048 A1 | 18-07-2018 |
| | | | ES | 2794557 T3 | 18-11-2020 |
| | | | JP | 6985252 B2 | 22-12-2021 |
| | | | JP | 2018531913 A | 01-11-2018 |
| | | | US | 2018243434 A1 | 30-08-2018 |
| | | | WO | 2017042210 A1 | 16-03-2017 |
| WO 2021213434 | A1 | 28-10-2021 | AU | 2021260639 A1 | 08-12-2022 |
| | | | BR | 112022021322 A2 | 06-12-2022 |
| | | | CN | 113527486 A | 22-10-2021 |
| | | | CN | 115427452 A | 02-12-2022 |
| | | | WO | 2021213434 A1 | 28-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012047724 A

**Non-patent literature cited in the description**

- **KABAT et al.** Sequences of Proteins of Immunological Interest. Public Health Service, National Institutes of Health, 1991
- **LEFRANC M.-P.** Unique database numbering system for immunogenetic analysis. *Immunology Today,* 1997, vol. 18, 509
- **LEFRANC M.-P.** The IMGT unique numbering for Immunoglobulins, T cell receptors and Ig-like domains. *The Immunologist,* 1999, vol. 7, 132-136
- **RIECHMANN, L. et al.** *Nature,* 1988, vol. 332, 323-327
- **NEUBERGER, M. S. et al.** *Nature,* 1985, vol. 314, 268-270
- **CONILH.** Exatecan antibody drug conjugates based on a hydrophilic polysarcosine drug-linker platform. *Pharmaceuticals,* 2021, vol. 14, 247